# EUROPEAN PATENT APPLICATION

(11) **EP 3 868 369 A1**
(43) Date of publication of application: **25.08.2021**
(21) Application number: 20158787.0
(22) Date of filing: 21.02.2020
(51) Int. Cl.: A61K 31/18, A61K 31/00, A61K 45/06, A61P 35/00

(54) **CXCL8 INHIBITOR AND PHARMACEUTICAL COMPOSITION THEREOF FOR USE IN THE TREATMENT OF CANCER-RELATED FATIGUE**

(71) Applicant: Dompe' Farmaceutici S.P.A., 20122 Milan (IT)
(72) Inventor: ALLEGRETTI, Marcello, 67100 L'Aquila (IT); RUFFINI, Pier Adelchi, 20122 Milano (IT)
(74) Representative: Mauri, Elisabetta Maria Ester

(57) **Abstract**

The present invention relates to a CXCL8 inhibitor and a pharmaceutical composition thereof, for use in the treatment of cancer-related fatigue in a cancer patient.

## Description

### FIELD OF THE INVENTION

The present invention relates to a CXCL8 inhibitor, such as reparixin, and a pharmaceutical composition thereof for use in the treatment of cancer-related fatigue, said fatigue being either related to cancer or to an anti-tumoral treatment, such as chemotherapy, radiotherapy or biological therapy.

### STATE OF THE ART

Cancer-related fatigue (CRF) is one of the most common symptoms experienced by cancer patients, occurring in more than 60% of cancer patients and more than 80% of those receiving cancer treatment. Cancer-related fatigue is reported to be the single most distressing symptom with the greatest negative impact on quality of life. It starts before diagnosis and persists for months or years after treatment completion.

Cancer treatments such as chemotherapy, radiation therapy or radiotherapy, and biologic therapy can cause or worsen fatigue in cancer patients. Fatigue is also a common symptom of some types of cancer.

The National Comprehensive Cancer Network (NCCN) defines cancer-related fatigue (CRF) as "a persistent, subjective sense of tiredness related to cancer or its treatment that interferes with usual functioning."

In the past, the terms "asthenia" and "weakness" were used to describe a subjective sensation of tiredness, while the specific term "fatigue" was used to describe a symptom of tiredness precipitated by effort. In particular, the term "fatigue" was used to refer to weariness or exhaustion resulting from physical or mental exertion, while "asthenia" was used to refer to weariness or exhaustion without physical or mental exertion.

However, the above terms are now used in the same context. The term "fatigue" has gained a more widespread acceptance in the medical literature and is preferentially used in the National Cancer Institute toxicity grading scale that covers generalized weakness. To describe fatigue, health professionals may use terms such as asthenia, lassitude, prostration, lack of energy and weakness. Patients describe fatigue as feeling tired, weak, worn-out, heavy, slow, or that they have no energy or get-up-and-go.

International Classification of Diseases (ICD-10) has put forward criteria for identifying cancer related fatigue (Cella D et al, J Clin Oncol. 2001;19:3385-91). This has been set to define the fatigue syndrome in all stages of cancer, from ongoing treatment to advanced stages of the disease as well as to survivorship.

For the purpose of the present description and claims, the term "fatigue" is used to include all the above terms and symptoms related to cancer or cancer treatment.

Clinicians, patients, family members, and researchers are increasingly recognizing that CRF represents a significant consequence of cancer and its treatment and requires clinical attention and intervention. Fatigue is one of the most frequently anticipated adverse effects of cancer treatment: 95% of patients who are scheduled to receive chemotherapy, radiotherapy or biological therapy expect to experience some degree of fatigue during their treatment. Reported incidence rates for CRF in the clinical trial setting tend to be in the range of 70%-80%. (M.Hofman et al, The Oncologist, 2007, vol. 12, pages 4-10).

Evidence-based treatments for CRF are scarce. Various interventions have been studied, including pharmacologic agents for symptom palliation (i.e., psychostimulants or steroids) and those that target possible etiologies (i.e., serotonin reuptake inhibitors) (J.M. Brell, L.W. Jones, in Abeloff's Clinical Oncology (Fifth Edition), 2014, pages 676-681). However, these are often not effective and many patients with cancer still note fatigue after treatment.

Some studies over the past few years have focused on a class of central nervous stimulants, which are widely used to target sleepiness. The most promising drug was considered modafimil, (Provigil), but a recent study showed the drug to have no benefit over placebo, although interestingly, both treatments brought about clinically significant changes in CRF (A.Spathis et al., Journal of Clinical Oncology, 2014, vol. 32, no. 18, pages 1882-1888).

Despite the efforts spent in the research of an effective treatment, CRF presently remains a concern for cancer patients. Accordingly, there is a persistent need for a new and more effective medication and treatment for adequate control of cancer-related fatigue.

### SUMMARY OF THE INVENTION

The applicant has identified a new treatment able to effectively control cancer-related fatigue.

In particular, the applicant has surprisingly found that the administration of a CXCL8 inhibitor, such as reparixin, to cancer patients is able to provide a consistent reduction in the incidence and severity of fatigue.

In particular, the applicant has found that in a multicentre, double-blind placebo-controlled clinical trial aimed to evaluate the progression-free survival of cancer patients treated by paclitaxel with and without the addition of reparixin, there was a significantly lower incidence and severity of treatment-related fatigue in the group of subjects treated with paclitaxel plus reparixin compared with the group of subjects treated with paclitaxel and placebo.

Accordingly, in a first aspect, the present invention relates to a CXCL8 inhibitor for use in the treatment of cancer-related fatigue in a cancer patient, more in particular of cancer-related-fatigue caused by an anti-tumoral treatment, preferably chemotherapy, radiotherapy or biological therapy.

In a second aspect, the present invention relates to a pharmaceutical composition comprising a CXCL8 inhibitor and at least one inert pharmaceutically acceptable excipient, for use in the treatment of cancer-related fatigue, more in particular of cancer-related fatigue caused by an anti-tumoral treatment, preferably chemotherapy, radiotherapy or biological therapy.

In a third aspect, the present invention relates to a method of treating cancer-related fatigue in a cancer patient, comprising administering to the subject a CXCL8 inhibitor.

### DETAILED DESCRIPTION OF THE INVENTION

A first aspect of the present invention relates to a CXCL8 inhibitor for use in the treatment of cancer-related fatigue in a cancer patient.

According to a preferred embodiment, said patient is undergoing an anti-tumoral treatment, preferably chemotherapy, radiotherapy or biological therapy, more preferably chmiotherapy.

Preferably, according to this embodiment, said cancer related fatigue is caused or worsened by said anti-tumoral treatment practiced on the patient, preferably chemotherapy, radiotherapy or biological therapy, more preferably chmiotherapy.

The term "CXCL8-inhibitor" according to the present application refers to any compound able to inhibit, partially or totally, the biological activity of CXCL8. Such a compound can act by decreasing the expression of CXCL8 or of its receptor(s) or by inhibiting the triggering of the intracellular signaling activated by the CXCL8 receptor(s). It is preferred that said CXCL8 activity inhibitor is able to inhibit at least 60%, preferably at least 70%, more preferably at least 80%, even more preferably at least 90% of PMNs chemotaxis induced by an optimal concentration of CXCL8 (1nM) at a concentration equal or below 500 nM, preferably below 100 nM.

According to a preferred embodiment, the CXCL8 inhibitor according to the present invention inhibits the activity of CXCL8 mediated by CXCR1 receptor or mediated by both CXCR1 and CXCR2 receptors. Preferably, according to this embodiment, said CXCL8 inhibitor is a CXCL8 receptor(s) inhibitor, which inhibits binding of CXCL8 to its receptor(s) or the intracellular signaling activated by the binding of CXCL8 to its receptor(s). Preferably, said CXCL8 receptor inhibitor is either an allosteric inhibitor or an orthosteric antagonist of CXCR1 receptor or of both CXCR1 and CXCR2 receptors. More preferably, the CXCL8 receptor(s) inhibitor according to the invention has an ICso value towards CXCR1 receptor in the low nanomolar range, preferably in the range 0.02-5 nanomolar.

According to a preferred embodiment, also in combination with any of the preceding embodiment, said CXCL8 inhibitor is selected from small molecular weight molecules, peptides and antibodies, more preferably it is a small molecular weight molecule.

CXCL8 inhibitors according to the above definition, able to inhibit the activity of CXCL8 mediated by CXCR1 receptor or mediated by both CXCR1 and CXCR2 receptors, are well known in the art.

To date, several CXCL8 inhibitors, such as small molecules, peptides and antibodies, have been disclosed, many of which are currently under undergoing clinical trials or are used in therapy. (Jie Jack, Expert Opinion Ther. Patents, 2001, 11(12), Chao J. et al., Bioorganic & Medicinal Chemistry Letters 17, 2007, p. 3778-3783, Busch-Petersen J. Current Topics in Medicinal Chemistry, 2006, 6, p. 1345-135, Allegretti et al, Immunology Letters 2012, Vol. 145, p. 68-78).

Preferred CXCL8 inhibitors according to the present invention are disclosed in WO2000/024710A1 and WO2005/090295, that also disclose their method of synthesis, their activity as CXCL8 inhibitors as well as their use as inhibitors of neutrophils chemotaxis and degranulation induced by CXCL8 and in the treatment of CXCL8 dependent pathologies.

Among these, CXCL8 inhibitors particularly preferred in the present invention are those of the following formula (I): 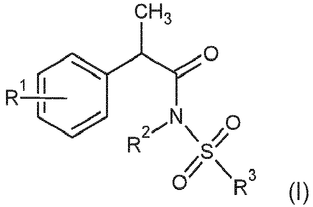 wherein
R¹ is selected from a linear or branched C₁-C₆ alkyl, benzoyl, phenoxy, and trifluoromethanesulfonyloxy;
R² is selected from hydrogen atom and linear or branched C₁-C₃ alkyl; and
R³ is a linear or branched C₁-C₆ alkyl or trifluoromethyl,
or a pharmaceutically acceptable salt thereof.

Preferably, R¹ is selected from benzoyl, isobutyl, and trifluoromethanesulfonyloxy. More in particular, R¹ is preferably linked to the phenyl ring in 3- or 4-position. According to the most preferred embodiment, R¹ is 3-benzoyl, 4-isobutyl or 4-trifluoromethanesulfonyloxy. Preferably, R² is selected from hydrogen atom or methyl.

Preferably, R³ is selected from linear or branched C₁-C₆ alkyl, more preferably from linear of branched C₁-C₃ alkyl. According to the most preferred embodiment, R³ is methyl.

Peferably, the chiral carbon of the compounds of formula (I) is in the RS or R configuration, more preferably it is in the R configuration.

Particularly preferred among said CXCL8 inhibitors of formula (I) are:
- 2-(4-isobutylphenyl)propionyl methansulfonamide, preferably R-(-)-2-(4-isobutylphenyl)propionyl methansulfonamide (also known as reparixin) and pharmaceutically acceptable salts thereof, preferably the lysine salt thereof, and
- 2-(4-trifluoromethanesulfonyloxy)phenyl]-N-methanesulfonyl propionamide, preferably R(-)-2-(4-trifluoromethanesulfonyloxy)phenyl]-N-methanesulfonyl propionamide (also known as ladarixin or DF2156A) and pharmaceutically acceptable salts thereof, in particular the sodium salt thereof.

Also, other preferred CXCL8 inhibitors according to the present invention are those disclosed in WO2010/031835, that also disclose their method of synthesis, their activity as CXCL8 inhibitors as well as their use as inhibitors of neutrophils chemotaxis and degranulation induced by CXCL8 and in the treatment of CXCL8 dependent pathologies. Among these, CXCL8 inhibitors particularly preferred in the present invention are those of the following formula (II): 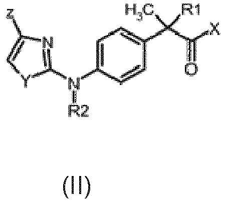 and pharmaceutically acceptable salts thereof, wherein:
R1 is hydrogen;
X is OH;
R2 is hydrogen or linear C1-C4 alkyl,
Y is a heteroatom selected from S, O and N,
Z is selected from linear or branched C1-C4 alkyl, linear or branched C1-C4 alkoxy, halo C1-C3 alkyl and halo C1-C3 alkoxy.

Peferably, the chiral carbon of the compounds of formula (II) is in the is in the RS or S configuration, more preferably in the S configuration.

Particularly preferred among said CXCL8 inhibitors of formula (II) is 2-(4-{[4-(trifluoromethyl)-1,3-thiazol-2-yl]amino}phenyl)propanoic acid, preferably (2S)-2-(4-{[4-(trifluoromethyl)-1,3-thiazol-2-yl] amino} phenyl) propanoic acid or the sodium salt thereof.

The most preferred CXCL8 inhibitors according to the present invention is R-(-)-2-(4-isobutylphenyl)propionyl methansulfonamide (also known as reparixin) and pharmaceutically acceptable salts thereof, preferably the lysine in situ salt thereof.

The CXCL8 inhibitor compounds of the present invention may form stable pharmaceutically acceptable acid or base salts with a pharmaceutically acceptable organic or inorganic acid or base, and in such cases administration of a compound as a salt may be appropriate.

Examples of acid addition salts include acetate, adipate, ascorbate, benzoate, benzenesulfonate, bicarbonate, bisulfate, butyrate, camphorate, camphorsulfonate, choline, citrate, cyclohexyl sulfamate, diethylenediamine, ethanesulfonate, fumarate, glutamate, glycolate, hemisulfate, 2-hydroxyethylsulfonate, heptanoate, hexanoate, hydrochloride, hydrobromide, hydroiodide, hydroxymaleate, lactate, malate, maleate, methanesulfonate, meglumine, 2-naphthalenesulfonate, nitrate, oxalate, pamoate, persulfate, phenylacetate, phosphate, diphosphate, picrate, pivalate, propionate, quinate, salicylate, stearate, succinate, sulfamate, sulfanilate, sulfate, tartrate, tosylate (p-toluenesulfonate), trifluoroacetate, and undecanoate.

Examples of base addition salts include ammonium salts; alkali metal salts such as sodium, lithium and potassium salts; alkaline earth metal salts such as aluminum, calcium and magnesium salts; salts with organic bases such as dicyclohexylamine salts and N-methyl-D-glucamine; and salts with amino acids such as arginine, lysine, ornithine, and so forth. Also, basic nitrogen-containing groups may be quaternized with such agents as: lower alkyl halides, such as methyl, ethyl, propyl, and butyl halides; dialkyl sulfates such as dimethyl, diethyl, dibutyl; diamyl sulfates; long chain halides such as decyl, lauryl, myristyl and stearyl halides; arylalkyl halides such as benzyl bromide and others. Nontoxic physiologically-acceptable salts are preferred, although other salts may be useful, such as in isolating or purifying the product.

The salts may be formed by conventional means, such as by reacting the free form of the product with one or more equivalents of the appropriate acid or base in a solvent or medium in which the salt is insoluble, such as for example water or ethanol, which is removed under vacuum or by freeze drying.

The present invention also includes the prodrugs, stereoisomers, isotope-labelled, for example deuterated, derivatives and enantiomers of the CXCL8 inhibitor compounds described above.

As used herein the term "prodrug" refers to an agent, which is converted into the parent drug in vivo by some physiological chemical process (e.g., a prodrug on being brought to the physiological pH is converted to the desired drug form). Prodrugs are often useful because, in some situations, they may be easier to administer than the parent drug. They may, for instance, be bioavailable by oral administration whereas the parent drug is not. The prodrug may also have improved solubility in pharmacological compositions over the parent drug. An example, without limitation, of a prodrug would be a compound of the present invention wherein it is administered as an ester (the "prodrug") to facilitate transmittal across a cell membrane where water solubility is not beneficial, but then it is metabolically hydrolysed once inside the cell where water solubility is beneficial. Prodrugs have many useful properties. For example, a prodrug may be more watersoluble than the ultimate drug, thereby facilitating intravenous administration of the drug. A prodrug may also have a higher level of oral bioavailability than the ultimate drug. After administration, the prodrug is enzymatically or chemically cleaved to deliver the ultimate drug in the blood or tissue.

Ester prodrugs of the CXCL8 inhibitor compounds disclosed herein are specifically contemplated. While not intending to be limiting, an ester may be an alkyl ester, an aryl ester, or a heteroaryl ester. The term alkyl has the meaning generally understood by those skilled in the art and refers to linear, branched, or cyclic alkyl moieties. C₁₋₆ alkyl esters are particularly useful, where alkyl part of the ester has from 1 to 6 carbon atoms and includes, but is not limited to, methyl, ethyl, propyl, isopropyl, n-butyl, sec-butyl, iso-butyl, t-butyl, pentyl isomers, hexyl isomers, cyclopropyl, cyclobutyl, cyclopentyl, cyclohexyl, and combinations thereof having from 1 to 6 carbon atoms.

Certain CXCL8 inhibitor compounds may exist in tautomeric forms, and this invention includes all such tautomeric forms of those compounds unless otherwise specified.

Unless otherwise stated, structures depicted herein are also meant to include all stereochemical forms of the structure; i.e., the R and S configurations for each asymmetric centre. Thus, single stereochemical isomers as well as enantiomeric and diastereomeric mixtures of the present compounds are within the scope of the invention. Thus, this invention encompasses each diastereomer or enantiomer substantially free of other isomers (>90%, and preferably >95%, free from other stereoisomers on a molar basis) as well as a mixture of such isomers.

Particular optical isomers can be obtained by resolution of the racemic mixtures according to conventional processes, e.g., by formation of diastereomeric salts, by treatment with an optically active acid or base. Examples of appropriate acids are tartaric, diacetyltartaric, dibenzoyltartaric, ditoluoyltartaric, and camphorsulfonic acid and then separation of the mixture of diastereomers by crystallization followed by liberation of the optically active bases from these salts. A different process for separation of optical isomers involves the use of a chiral chromatography column optimally chosen to maximize the separation of the enantiomers. Still another method involves synthesis of covalent diastereomers by reacting compounds of the invention with an optically pure acid in an activated form or an optically pure isocyanate. The synthesized diastereomers can be separated by conventional means such as chromatography, distillation, crystallization or sublimation, and then hydrolysed to deliver the enantiomerically pure compound. Optically active compounds of the invention can be obtained by using active starting materials. These isomers may be in the form of a free acid, a free base, an ester or a salt.

The term "chemotherapy" according to the present application refers to any cancer treatment using an antitumor chemotherapeutic agent (including but not limited to alkylating agents, anthracyclines, cytoskeletal disruptors (taxanes), antimetabolites, topoisomerase I and II inhibitors, kinase inhibitors, platinum-based agents, and vinca alkaloids) to kill cancer cells and reduce or eliminate the growth or proliferation of tumors. The expression "antitumor chemotherapeutic agent" according to the present application refers to any compound employed in the cancer therapy able to kill the cancer cells, such as for example, alkylating agents, anthracyclines, cytoskeletal disruptors (taxanes), antimetabolites, topoisomerase I and II inhibitors, kinase inhibitors, platinum-based agents, vinca alkaloids, and the like.

Chemotherapeutic agent are known in the art. A non-exhaustive compilation can be easily found at https://en.wikipedia.org/wiki/List of antineoplastic agents.

Useful examples of chemotherapeutic agents are (i) alkylating agents, such as, for example, cyclophosphamide, mechlorethamine, chlorambucil, melphalan, (ii) anthracyclines, such as for example, doxorubicin, epirubicin, mitoxantrone, pixantrone, losoxantrone, and daunorubicin, (iii) cytoskeletal disruptors (taxanes), such as, for example, paclitaxel, docetaxel, abraxane, and taxotere, (iv) antimetabolites, such as, for example, azacitidine, azathioprine, capecitabine, cladribine, cytarabine, doxifluridine, hydroxyurea, methotrexate; 6-mercaptopurine, 6-thioguanine, fludarabine phosphate, pentostatin, pemetrexed, 5-fluorouracil, 5-fluorodeoxyuridine, and gemcitabine, (v) topoisomerase I and II inhibitors, such as, for example, irinotecan, topotecan, etoposide, teniposide, tafluposide, (vi) kinase inhibitors, such as, for example, bortezomib, erlotinib, gefitinib, imatinib, vemurafenib, vismodegib, (vii) platinum-based agents, such as, for example, carboplatin, cisplatin, oxaliplatin, and (viii) vinca alkaloids, such as, for example, vinblastine, vincristine, vindesine, vinorelbine.

The term "radiotherapy" according to the present application refers to any cancer treatment using a source of high-energy radiation, including but not limited to X-rays, gamma rays, particle beams, proton beam therapy, and high-energy photon radiation, to kill cancer cells and reduce or eliminate the growth or proliferation of tumors, such as for example external radiation therapy (such as e.g., intraoperative radiotherapy and prophylactic cranial irradiation (PC), intensity-modulated radiation therapy (IMRT), stereotactic (or stereotaxic) radiosurgery, three-dimensional (3-D) conformal radiation therapy, Volumetric modulated arc therapy (VMAT), particle therapy, Auger therapy), internal radiation therapy (such as e.g., interstitial radiation therapy, intracavitary or intraluminal radiation therapy), and systemic radiation therapy (such as e.g., lodine-131 (¹³¹I), Radium-223 (²²³Ra), Strontium-89 (⁸⁹Sr), Phosphorus-32 (³²P), Yttrium-90 (⁹⁰Y) therapy).

The expression "biological therapy" according to the present application refers to a treatment of cancer that uses the immune system of the patient to kill the cancer cells, by targeting the cancer cells directly, for example through antibodies, or indirectly by inducing the immune system to attack the cancer cells or by making cancer cells easier for your immune system to recognize. A preferred biological therapy according to the invention is immunotherapy.

In a preferred embodiment of the present invention, the CXCL8 inhibitor is for use in cancer-related fatigue caused or worsened by treatment with a chemotherapeutic agent. Preferably, said chemotherapeutic agent is selected from cisplatin, paclitaxel, docetaxel, abraxane, taxotere, 5-fluorouracil, more preferably it is paclitaxel.

Typically, the CXCL8 inhibitor for use according to the first aspect of the invention is administered in the form of a pharmaceutical composition.

Accordingly, a second aspect of the present invention relates to a pharmaceutical composition comprising a CXCL8 inhibitor and at least one inert pharmaceutically acceptable excipient, for use in the treatment of cancer-related fatigue, more in particular of cancer-related fatigue caused by an anti-tumoral treatment, such as chemotherapy or radiotherapy, as above defined.

Preferably, the pharmaceutical composition of the present invention is prepared in suitable dosage forms comprising an effective amount of a CXCL8 inhibitor, a salt thereof with a pharmaceutically acceptable organic or inorganic acid or base, or a prodrug thereof, and at least one inert pharmaceutically acceptable excipient.

The administration of the pharmaceutical composition of the present invention to a patient is in accord with known methods and may comprise from one to several oral administrations per day (for example, two times a day (BID) or four times a day (QID)), parenteral administration (including intravenous, intraperitoneal, intracerebral, intrathecal, intracranial, intramuscular, intraarticular, intrasynovial, intrasternal, intraocular, intraarterial, subcutaneous, intracutaneous or intralesional injection or infusion techniques), topical, buccal and suppository administration, or by sustained release systems among other routes of administration.

In the present description and in the following claims, the wording "effective amount" means an amount of a compound or composition which is sufficient enough to significantly and positively modify the symptoms and/or conditions to be treated (e.g., provide a positive clinical response). The effective amount of an active ingredient for use in a pharmaceutical composition will vary with the particular condition being treated, the severity of the condition, the duration of the treatment, the nature of concurrent therapy, the particular active ingredient(s) being employed, the particular pharmaceutically-acceptable excipient(s)/carrier(s) utilized, and like factors within the knowledge and expertise of the attending physician.

As described herein, the pharmaceutical composition of the present invention comprises a CXCL8 inhibitor together with a pharmaceutically acceptable excipient, which, as used herein, includes any and all solvents, diluents, or other vehicle, dispersion or suspension aids, surface active agents, isotonic agents, thickening or emulsifying agents, preservatives, solid binders, lubricants and the like, as suited to the particular dosage form desired.

Some examples of materials which can serve as pharmaceutically acceptable excipient include, but are not limited to, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatine; talc; excipients such as cocoa butter and suppository waxes; oils such as peanut oil, cottonseed oil; safflower oil; sesame oil; olive oil; corn oil and soybean oil; glycols; such a propylene glycol; esters such as ethyl oleate and ethyl laurate; agar; buffering agents such as magnesium hydroxide and aluminum hydroxide; alginic acid; pyrogen-free water; isotonic saline; sterilized water; Ringer's solution; buffered saline; dextrose solution; maltodextrin solution; ethyl alcohol; and phosphate buffer solutions. Moreover, the composition of the present invention may be formulated into inhalable or injectable dosage forms such as solutions, suspensions, and emulsions by further adding diluents, dispersants, and surfactants.

Further, the composition of the present invention may be suitably formulated using appropriate methods known in the art or by the method disclosed in Remington's Pharmaceutical Science (recent edition), Mack Publishing Company, Easton Pa.

The terms "pharmaceutically acceptable" and "physiologically acceptable" are intended to define, without any particular limitation, any material suitable for preparing a pharmaceutical composition to be administered to a living being.

The dosage forms can also contain other traditional ingredients such as: preservatives, stabilizers, surfactants, buffers, salts for regulating osmotic pressure, emulsifiers, sweeteners, colorants, flavourings and the like.

Also specific dosage and treatment regimens for any particular patient will depend upon a variety of factors, including for example the activity and half life of the specific compound employed, the age, body weight, general health status, sex, diet, severity and course of the disease.

The amount of a CXCL8 inhibitor or the pharmaceutically acceptable salt thereof in the pharmaceutical composition of the present invention can vary over a wide range depending on known factors, for example, the molecule used, the severity of the disease, the patient's body weight, the dosage form, the chosen route of administration, and the number of administrations per day. However, a person skilled in the art can determine the optimum amount in easily and routinely manner.

For example, whent the CXCL8 inhibitor is reparixin the amount of said CXCL8 inhibitor or the pharmaceutically acceptable salt thereof in the pharmaceutical composition of the present invention will be such as to ensure a dosage per single administration of from 400 to 1400 mg, preferably from to 800-1200 mg, even more preferably 1200 mg, to be administered three times a day.

As the skilled artisan will appreciate, lower or higher doses than those recited above may be required. Specific dosage and treatment regimens for any particular patient will depend upon a variety of factors, including the activity of the specific compound employed, the age, body weight, general health status, sex, diet, time of administration, rate of excretion, drug combination, the severity and course of the disease, and the patient's disposition to the disease and the judgment of the treating physician.

The dosage forms of the pharmaceutical composition of the present invention can be prepared by techniques that are familiar to a pharmaceutical chemist, and comprise mixing, granulation, compression, dissolution, sterilization and the like.

In a third aspect, the present invention relates to a method of treating cancer-related fatigue in a cancer patient, comprising administering to the patient a CXCL8 inhibitor as above defined.

The above method preferably comprises the steps of i) identifying a cancer patient suffering from cancer-related fatigue, and ii) administering to said patient a composition comprising a CXCL8 inhibitor, a salt thereof with a pharmaceutically acceptable organic or inorganic acid or base, or a prodrug thereof.

According to a preferred embodiment, said patient is undergoing an anti-tumoral treatment, preferably chemotherapy, radiotherapy or biological therapy.

Preferably, according to this embodiment, said cancer related fatigue is caused or worsened by said anti-tumoral treatment practiced on the patient Preferably, in the method according to the invention, said CXCL8 inhibitor is administered in form of a pharmaceutical composition, as above defined.

In accordance with the diferent aspects of the invention, preferably the CXCL8 inhibitor is administered simultaneously or sequentially with an anti-tumoral treatment.

The invention will be further described in the following examples, which do not limit the scope of the invention described in the claims.

### EXPERIMENTAL PART

A randomized, double-blind, placebo-controlled phase 2 study was conducted to evaluate the activity of paclitaxel and reparixin compared to paclitaxel alone as front-line therapy for Metastatic Triple-Negative Breast Cancer (FRIDA).

The study was a two arm, phase 2 study to evaluate the efficacy of the administration of paclitaxel and reparixin compared to paclitaxel and placebo in metastatic TNBC patients. The study was conducted with 56 sites actively screening patients in the United States of America, Italy, France, Belgium, Spain, Czech Republic and Poland.

The first patient was enrolled on 29 July 2015 and the last patient was enrolled on 30 July 2018. The data cut-off for main analysis was 20 February 2019. The trial was terminated after 123 patients were randomized.

The primary objective of the study was to evaluate progression-free survival (PFS) (defined as the number of days between the date of randomization and the date of clinical disease progression (PD) according to Response Evaluation Criteria in Solid Tumors (RECIST) criteria version 1.1, as assessed by Independent Radiology Review, or death for any cause, whichever occurred first) in patients with metastatic triple negative breast cancer (TNBC) treated with paclitaxel and orally administered reparixin compared to paclitaxel alone.

Two sub-groups of patients were studied: newly diagnosed metastatic patients and patients that had relapsed following a prior (neo)adjuvant chemotherapy regimen. Amongst other inclusion criteria, to be able to participate in the study, subjects were to have a Zubrod/ECOG Performance Status of 0 or 1 (0 = Fully active; no performance restrictions 1 = Strenuous physical activity restricted; fully ambulatory and able to carry out light work).

The secondary objectives of the study were:
- to determine overall survival (OS),
- to evaluate objective response rates (ORR),
- to determine median PFS (mPFS),
- to assess the safety of paclitaxel and orally administered reparixin (referred to as combination treatment).

The exploratory objectives of the study were:
- to determine time to new metastasis (new lesions at existing or new sites),
- to determine the proportion of patients progressing with new metastatic lesions,
- to compare the incidence and severity of peripheral neuropathy between the two treatment groups,
- to evaluate cancer stem cells (CSCs) in metastatic tissue,

The study comprised two treatment groups:
- Group 1: paclitaxel 80 mg/m² intravenous (i.v.) (Days 1, 8, and 15 of 28-day cycle) + reparixin oral tablets 1200 mg three times a day (t.i.d.) continuing from Day 1 to Day 21.
- Group 2: paclitaxel 80 mg/ m² i.v. (Days 1, 8, and 15 of 28-day cycle) + placebo oral tablets 1200 mg t.i.d. continuing from Day 1 to Day 21.

There was no limit to study treatment duration and patients remained in the study until disease progression, withdrawal of consent or unacceptable toxicity, whichever occurred first.

Tumor response and/or progression assessments were performed and documented every eight weeks according to RECIST criteria version 1.1. Metastatic tissue samples were analyzed for evaluation of CD24- CD44+ and aldehyde dehydrogenase positive (ALDH+) CSCs.

Investigators reported any adverse events experienced by study patients according to the NCI-CTCAE version 4.03. Toxicity was defined according to the grading scale for each parameter. According to this scale, the presence and intensity of fatigue is defined as follows:
- Grade 1 : Fatigue relieved by rest
- Grade 2 : Fatigue not relieved by rest, limiting instrumental ADL
- Grade 3 : Fatigue not relieved by rest, limiting self care ADL
where instrumental ADL refers to preparing meals, shopping for groceries or clothes, using the telephone, managing money, and the like and self care ADL refer to bathing, dressing and undressing, feeding self, using the toilet, taking medications, but not bedridden.

The results of patient evaluation are summarized in the following Tables. The distributions of fatigue as a treatment emergent adverse event (TEAE) irrespective of relationship with study treatment were compared among treatment groups using the Chi-squared test (Table 1).

**TABLE 1**

| Fatigue | Group 1 | Group 2 | Both groups | Chi-squared Pvalue |
|---|---|---|---|---|
| No | 50 (82.0%) | 34 (56.7%) | 84 (69.4%) | 0.003 |
| Yes | 11 (18.0%) | 26 (43.3%) | 37 (30.6%) | |

The results of the study surprisingly showed that there was a lower incidence of treatment-related fatigue in Group 1 (paclitaxel + reparixin), with only 11 (18%) subjects experiencing fatigue, compared to 26 (43.3%) subjects in the control Group 2.

The difference between the reparixin Group 1 and placebo Group 2 for fatigue was statistically significant (P=0.003)

The distributions of fatigue as TEAE judged by investigators under double-blind conditions as at least possibly related to study treatment (i.e., paclitaxel + reparixin or paclitaxel + placebo) also were compared among treatment groups using the Chi-squared test (Table 2)

**TABLE 2**

| Fatigue | Group 1 | Group 2 | Both groups | Chi-squared Pvalue |
|---|---|---|---|---|
| No | 54 (88.5%) | 43 (71.7%) | 97 (80.2%) | 0.020 |
| Yes | 7 (11.5%) | 17 (28.3%) | 24 (19.8%) | |

The results of the study surprisingly showed that there was a lower incidence of treatment-related fatigue in Group 1 (paclitaxel + reparixin), with only 7 (11.5%) subjects experiencing fatigue, compared to 17 (28.3%) subjects in the control Group 2.

The difference between the reparixin Group 1 and placebo Group 2 for treatment-related fatigue was statistically significant (P=0.020).

Furthermore, in the patients of the two groups were fatigue was present, this was classidied with lower intensity in Group 1 compared to Group 2. In particular, all patients experiencing fatigue in Group 1 were reported with mild and moderate (NCI-Common Toxicity Criteria Grade 1-2) severity, whereas in the control Group 2 two subjects (3.3%) were reported as having experienced severe (Grade 3) treatment-related fatigue.

This evidence demonstrates that reparixin had a beneficial effect on incidence and severity of cancer-related fatigue, a common cancer treatment symptom/adverse events.

## Claims

1. A CXCL8 inhibitor, a salt thereof with a pharmaceutically acceptable organic or inorganic acid or base, or a prodrug thereof, for use in the treatment of cancer-related fatigue.

2. The CXCL8 inhibitor for use as claimed in claim 1, wherein said CXCL8 inhibitor is a CXCR1 inhibitor or a dual CXCR1 and CXCR2 inhibitor.

3. The CXCL8 inhibitor for use as claimed in claim 1 or 2, wherein said CXCL8 inhibitor is a small molecule, antibody or peptide.

4. The CXCL8 inhibitor for use as claimed in claims 1 to 3, wherein said CXCL8 inhibitor is a small molecular weight molecule represented by the following formula (I): 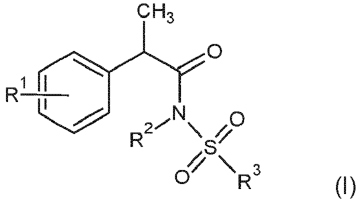 wherein
R¹ is selected from a linear or branched C₁-C₆ alkyl, benzoyl, phenoxy, and trifluoromethanesulfonyloxy;
R² is selected from hydrogen and linear or branched C₁-C₃ alkyl; and R³ is a linear or branched C₁-C₆ alkyl or trifluoromethyl,
a salt thereof with a pharmaceutically acceptable organic or inorganic acid or base, or a prodrug thereof.

5. The CXCL8 inhibitor for use as claimed in claims 1 to 4, wherein said CXCL8 inhibitor is selected from:
- 2-(4-isobutylphenyl)propionyl methansulfonamide, preferably R-(-)-2-(4-isobutylphenyl)propionyl methansulfonamide and pharmaceutically acceptable salts thereof, preferably the lysine salt thereof, and
- 2-(4-trifluoromethanesulfonyloxy)phenyl]-N-methanesulfonyl propionamide, preferably R(-)-2-(4-trifluoromethanesulfonyloxy)phenyl]-N-methanesulfonyl propionamide and pharmaceutically acceptable salts thereof, in particular the sodium salt thereof.

6. The CXCL8 inhibitor for use as claimed in claims 1 to 5, wherein said CXCL8 inhibitor is R-(-)-2-(4-isobutylphenyl)propionyl methansulfonamide or a pharmaceutically acceptable salt thereof, preferably the lysine salt thereof.

7. The CXCL8 inhibitor for use as claimed in claims 1 to 3, wherein said CXCL8 inhibitor is represented by the following formula (II): 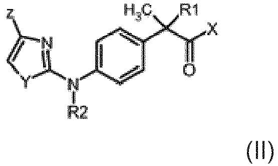 wherein:
R1 is hydrogen;
X is OH;
R2 is hydrogen or linear C1-C4 alkyl,
Y is a heteroatom selected from S, O and N,
Z is selected from linear or branched C1-C4 alkyl, linear or branched C1-C4 alkoxy, halo C1-C3 alkyl and halo C1-C3 alkoxy
a salt thereof with a pharmaceutically acceptable organic or inorganic acid or base, or a prodrug thereof.

8. The CXCL8 inhibitor for use as claimed in claim 7, wherein said CXCL8 inhibitor is 2-(4-{[4-(trifluoromethyl)-1,3-thiazol-2-yl]amino}phenyl)propanoic acid, preferably (2S)-2-(4-{[4-(trifluoromethyl)-1,3-thiazol-2-yl]amino}phenyl)propanoic acid or the sodium salt thereof.

9. The CXCL8 inhibitor for use as claimed in claims 1 to 8, wherein said patient is undergoing an antitumoral treatment and/or said cancer-related fatigue is caused or worsened by an anti-tumoral treatment.

10. The CXCL8 inhibitor for use as claimed in claims 1 to 9, wherein said anti-tumoral treatment is selected from chemotherapy, radiotherapy and biological therapy.

11. The CXCL8 inhibitor for use as claimed in claim 10, wherein said radiotherapy is selected from external radiation therapy (such as e.g., intraoperative radiotherapy and prophylactic cranial irradiation (PC), intensity-modulated radiation therapy (IMRT), stereotactic (or stereotaxic) radiosurgery, three-dimensional (3-D) conformal radiation therapy, Volumetric modulated arc therapy (VMAT), particle therapy, Auger therapy), internal radiation therapy (such as e.g., interstitial radiation therapy, intracavitary or intraluminal radiation therapy), and systemic radiation therapy (such as e.g., lodine-131 (¹³¹I), Radium-223 (²²³Ra), Strontium-89 (⁸⁹Sr), Phosphorus-32 (³²P), Yttrium-90 (⁹⁰Y) therapy).

12. The CXCL8 inhibitor for use as claimed in claim 10, wherein said chemotherapy is a antitumoral treatment with a chemotherapeutic agent selected from alkylating agents, anthracyclines, cytoskeletal disruptors (taxanes), antimetabolites, topoisomerase I and II inhibitors, kinase inhibitors, platinum-based agents, and vinca alkaloids.

13. The CXCL8 inhibitor for use as claimed in claim 12, wherein said chemotherapeutic agent is selected from (i) cyclophosphamide, mechlorethamine, chlorambucil, melphalan, (ii) doxorubicin, epirubicin, mitoxantrone, pixantrone, losoxantrone, daunorubicin, (iii) paclitaxel, docetaxel, abraxane, taxotere, (iv) azacitidine, azathioprine, capecitabine, cladribine, cytarabine, doxifluridine, hydroxyurea, methotrexate; 6-mercaptopurine, 6-thioguanine, fludarabine phosphate, pentostatin, pemetrexed, 5-fluorouracil, 5-fluorodeoxyuridine, gemcitabine, (v) irinotecan, topotecan, etoposide, teniposide, tafluposide, (vi) bortezomib, erlotinib, gefitinib, imatinib, vemurafenib, vismodegib, (vii) carboplatin, cisplatin, oxaliplatin, (viii) vinblastine, vincristine, vindesine, and vinorelbine, preferably, it is cisplatin, paclitaxel, docetaxel, abraxane, taxotere, 5-fluorouracil, more more preferably it is paclitaxel.

14. The CXCL8 inhibitor for use as claimed in claim 10, wherein said biological therapy is immunotherapy.

15. A pharmaceutical composition comprising (i) a CXCL8 inhibitor, a salt thereof with a pharmaceutically acceptable organic or inorganic acid or base, or a prodrug thereof, as defined in any one of claims 1 to 6, and (ii) at least one inert pharmaceutically acceptable excipient, for use in the treatment of cancer-related fatigue in a cancer patient.
